# EUROPEAN PATENT APPLICATION

(11) **EP 4 835 333 A2**
(43) Date of publication of application: **04.11.2026**
(21) Application number: 26205685.6
(22) Date of filing: 25.11.2020
(51) Int. Cl.: F16J 15/32

(54) **BIOPSY DEVICE CANNULA SEALS**

(30) Priority: 26.11.2019 US 201962940616 P
(62) Document number(s) of the earlier application(s) in accordance with Art. 76 EPC: 20828457.0 / 4 065 002
(71) Applicant: Hologic, Inc., Marlborough, MA 01752 (US)
(72) Inventor: GIRGENTI, John, Marlborough, 01752 (US); DEFREITAS, Kenneth F., Patterson, 12563 (US); BURCHFIELD, Brent, Marlborough, 01752 (US); GILLUM, Justin, Marlborough, 01752 (US)
(74) Representative: Carpmaels & Ransford LLP

(57) **Abstract**

A biopsy device includes an elongated housing having a manifold, an outer cannula partially and slidably disposed in the manifold, and an inner cannula partially and slidably disposed in a lumen of the outer cannula. The biopsy device further includes an outer cannula seal disposed between the manifold and the outer cannula, the outer cannula seal including an interference ring portion disposed adjacent an inner surface of the manifold, a beaded ring portion that contacts an outer surface of the outer cannula, and a flexible portion extending between the interference ring portion and the beaded ring portion.

## Description

### Related Application Data

The present disclosure claims priority to U.S. Provisional Patent Application No. 62/940,616, filed on November 26, 2019, under attorney docket number BSH.0170.01 PRO and entitled "BIOPSY DEVICE CANNULA SEAL," which is hereby incorporated by reference into the present application in its entirety, as though set forth in full. The subject matter of this application relates to the subject matter disclosed and described in U.S. Patent No. 10,022,110, which is hereby incorporated by reference into the present application in its entirety, as though set forth in full.

### Field

The present disclosure generally relates to fluid-tight seals used in biopsy device needle sets.

### Background

In the practice of diagnostic medicine, it is often necessary or desirable to perform a biopsy, or to sample selected tissue from a living patient for medical evaluation. Cytological and histological studies of the biopsy sample can then be performed as an aid to the diagnosis and treatment of disease. Biopsies can be useful in diagnosing and treating various forms of cancer, as well as other diseases in which a localized area of affected tissue can be identified.

Biopsies are routinely performed on tissue using a needle set. One known needle set includes an outer cannula having a pointed tip and a tissue receiving opening defined near its distal end, and an inner cannula having an open distal end surrounded by an annular cutting blade. The inner cannula is slidably disposed within the outer cannula so that it can close the tissue receiving opening, thereby cutting tissue prolapsing into the lumen of the outer cannula through the tissue receiving opening. Typically, a hub is connected to the proximal end of each needle. Such needle sets are used with or incorporated in various forms of biopsy devices, including both manual and motor driven biopsy devices.

Current needle sets include one or more O-rings to attempt to provide a fluid-tight seals between various surfaces. However, O-rings may not be able to maintain a fluid-tight seal, especially with movement of various needle set components (e.g., outer and/or inner cannulas) in contact with the O-rings. Further, lateral movement of the outer and/or inner cannulas may also compromise the fluid-tight seal. Moreover, O-rings generate seals using frictional fits (e.g., against outer and/or inner cannulas). As such, when those outer and/or inner cannulas move relative to the O-rings, the O-rings exert a drag on the outer and/or inner cannulas. This drag reduces the efficiency of the biopsy devices into which the needle sets are incorporated. The drag is exacerbated with large longitudinal displacements of the outer and/or inner cannulas during biopsy. The limitations when using O-rings in needle sets as described above also apply to other conventional seal such as "X" profile and lip type seals.

### Summary

In accordance with one embodiment, a biopsy device includes an elongated housing having a manifold, an outer cannula partially and slidably disposed in the manifold, and an inner cannula partially and slidably disposed in a lumen of the outer cannula. The biopsy device further includes an outer cannula seal disposed between the manifold and the outer cannula, the outer cannula seal including an interference ring portion disposed adjacent an inner surface of the manifold, a beaded ring portion that contacts an outer surface of the outer cannula, and a flexible portion extending between the interference ring portion and the beaded ring portion.

In one or more embodiments, the outer cannula seal is configured to allow longitudinal movement of the outer cannula relative to the manifold while maintaining a fluid-tight seal therebetween. The outer cannula seal may have a partial conical shape or a V-shaped cross-section. The biopsy device may also include a manifold cap coupled to a distal end of the manifold, where the manifold cap and the manifold together define an annular space adjacent to the distal end of the manifold, and where the interference ring portion of the outer cannula seal is at least partially disposed in the annular space. The interference ring portion of the outer cannula seal may form an interference fit between the inner surface of the manifold and an inner surface of the manifold cap. The interference ring portion of the outer cannula seal may define a distally facing annular detent configured to engage a proximally facing annular lip of the manifold cap.

In one or more embodiments, the flexible portion of the outer cannula seal is biased to cause the beaded ring portion to apply a force against the outer surface of the outer cannula to create a fluid-tight seal between the beaded ring portion and the outer surface of the outer cannula. The flexible portion of the outer cannula seal may be configured to allow the interference ring portion and beaded ring portion to move longitudinally relative to each other while maintaining a fluid-tight seal between the outer cannula and the manifold. The outer cannula seal may include an ethylene propylene diene monomer ("EPDM") polymer. The outer cannula seal may be manufactured using a mold-forming process.

In accordance with one embodiment, a biopsy device includes an elongated housing having a seal sleeve, an outer cannula, an outer cannula hub coupled to a proximal end of an outer cannula, the outer cannula hub being partially and slidably disposed in the seal sleeve, and an inner cannula partially and slidably disposed in a lumen of the outer cannula. The biopsy device further includes an inner cannula seal disposed between the seal sleeve and the inner cannula, the inner cannula seal including an interference ring portion disposed adjacent an inner surface of the seal sleeve, a beaded ring portion that contacts an outer surface of the inner cannula, and a flexible portion extending between the interference ring portion and the beaded ring portion.

In one or more embodiments, the inner cannula seal is configured to allow longitudinal movement of the inner cannula relative to the seal sleeve while maintaining a fluid-tight seal therebetween. The inner cannula seal may have a partial conical shape or a J-shaped cross-section. A proximal end of the outer cannula hub may define an annular groove adjacent a distal end of the seal sleeve, and where the interference ring portion of the inner cannula seal is at least partially disposed in the annular groove. The interference ring portion of the inner cannula seal may form an interfere fit within the annular groove. The inner cannula seal may also include a middle beaded ring portion extending from on an outer surface of the flexible portion.

In one or more embodiments, the flexible portion of the inner cannula seal is biased to cause the beaded ring portion to apply a force against the outer surface of the inner cannula to create a fluid-tight seal between the beaded ring portion and the outer surface of the inner cannula. The flexible portion of the inner cannula seal may be configured to allow the interference ring portion and the beaded ring portion to move longitudinally relative to each other while maintaining a fluid-tight seal between the inner cannula and the seal sleeve. The inner cannula seal may include an ethylene propylene diene monomer ("EPDM") polymer. The inner cannula seal may be manufactured using a mold-forming process.

In accordance with still another embodiment, a biopsy device includes an elongated housing including a saline return fitting, an outer cannula partially and slidably disposed in the elongated housing, and an inner cannula, where the inner cannula is partially and slidably disposed in each of a lumen of the outer cannula and a lumen of the saline return fitting. The biopsy device further includes an inner cannula seal disposed between an inner wall of the saline return fitting and the inner cannula, the inner cannula seal including an interference ring portion disposed adjacent an inner surface of the saline return fitting, a beaded ring portion that contacts an outer surface of the inner cannula, and a flexible portion that extends between the interference ring portion and the beaded ring portion.

In one or more embodiments, the inner cannula seal is configured to allow longitudinal movement of the inner cannula relative to the saline return fitting while maintaining a fluid-tight seal therebetween. The inner cannula seal may have a partial conical shape or a J-shaped cross-section. The biopsy device may also include a saline return fitting cap coupled to a distal end of the saline return fitting, where a proximal end of the saline return fitting cap defines an annular groove adjacent the distal end of the saline return fitting, and where the interference ring portion of the inner cannula seal is at least partially disposed in the annular groove. The interference ring portion of the inner cannula seal may be at least partially disposed in the annular groove. The inner cannula seal may also include a middle beaded ring portion extending from an outer surface of the flexible portion.

In one or more embodiments, the flexible portion of the inner cannula seal is biased to cause the beaded ring portion to apply a force against the outer surface of the inner cannula to create a fluid-tight seal between the beaded ring portion and the outer surface of the inner cannula. The flexible portion of the inner cannula seal may be configured to allow the interference ring portion and the beaded ring portion to move longitudinally relative to each other while maintaining a fluid-tight seal between the inner cannula and the saline return fitting. The inner cannula seal may include an ethylene propylene diene monomer ("EPDM") polymer. The inner cannula seal may be manufactured using a mold forming process.

Other and further aspects and features of embodiments of the disclosed inventions will become apparent from the ensuing detailed description in view of the accompanying figures.

### Brief Description of the Drawings

The drawings illustrate the design and utility of embodiments of the disclosed inventions, in which similar elements are referred to by common reference numerals. These drawings are not necessarily drawn to scale. In order to better appreciate how the above-recited and other advantages and objects are obtained, a more particular description of the embodiments will be rendered, which are illustrated in the accompanying drawings. These drawings depict only typical embodiments of the disclosed inventions and are not therefore to be considered limiting of its scope.
Figure 1 is a side cross-sectional view of a biopsy device for use with a two-part biopsy device constructed according to one embodiment of the disclosed inventions.
Figures 2-4 are detailed side cross-sectional views of the biopsy device depicted in Figure 1.
Figures 5-8 are respective side, cross-sectional, perspective, front, and side views of a cannula seal constructed according to another embodiment of the disclosed inventions.
Figures 9-12 are respective side, cross-sectional, perspective, front, and side views of a cannula seal constructed according to yet another embodiment of the disclosed inventions.

### Detailed Description of the Illustrated Embodiments

For the following defined terms, these definitions shall be applied, unless a different definition is given in the claims or elsewhere in this specification.

All numeric values are herein assumed to be modified by the term "about," whether or not explicitly indicated. The term "about" generally refers to a range of numbers that one of skill in the art would consider equivalent to the recited value (i.e., having the same function or result). In many instances, the terms "about" may include numbers that are rounded to the nearest significant figure.

The recitation of numerical ranges by endpoints includes all numbers within that range (e.g., 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

Various embodiments of the disclosed inventions are described hereinafter with reference to the figures. It should be noted that the figures are not drawn to scale. It should also be noted that the figures are only intended to facilitate the description of the embodiments. They are not intended as an exhaustive description of the invention or as a limitation on the scope of the invention, which is defined only by the appended claims and their equivalents. In addition, an illustrated embodiment of the disclosed inventions needs not have all the aspects or advantages shown. An aspect or an advantage described in conjunction with a particular embodiment of the disclosed inventions is not necessarily limited to that embodiment and can be practiced in any other embodiments even if not so illustrated. In order to better appreciate how the above-recited and other advantages and objects are obtained, a more particular description of the embodiments will be rendered, which are illustrated in the accompanying drawings. These drawings depict only typical embodiments of the disclosed inventions and are not therefore to be considered limiting of its scope.

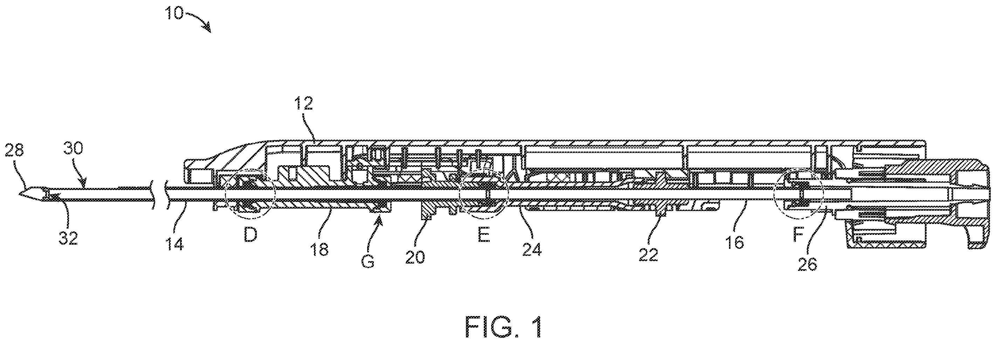

Figure 1 depicts a needle set 10 for use with a two-part biopsy device in a longitudinal cross-sectional view to allow depiction of internal components. The needle set 10 is a "disposable portion" of a two-part biopsy device. The second part of the two-part biopsy device (not shown) is a "reusable portion," which includes the drive mechanisms for various components of the needle set 10. The needle set 10 is configured to be discarded after a single use, while the reusable portion is configured to be cleaned after each use and used in subsequent biopsies. As such, the disposable needle set 10, which comes into contact with tissue during a biopsy, is discarded after the biopsy, while the reusable portion, which can be isolated from tissue during a biopsy, is cleaned and reused. The drive mechanisms in the reusable portion are typically more costly than the components of the needle set 10. Therefore, reusing the reusable portion and disposing of the needle set 10 reduces the cost of biopsies. Additional details regarding exemplary two-part biopsy devices and reusable portions are described in U.S. patent number 10,022,110, which has been previously incorporated by reference herein.

The needle set 10 includes a housing 12, an outer cannula 14, an inner cannula 16, a manifold 18, an outer cannula hub 20, an inner cannula hub 22, a seal sleeve 24, and a saline return fitting 26. The outer cannula 14 has a distal tissue piercing tip 28 and a tissue receiving opening (or "aperture") 30 defined near its distal end adjacent the distal tissue piercing tip 28. The inner cannula 16 has an open distal end 32 surrounded by an annular cutting blade. The inner cannula 16 is partially and slidably disposed within the outer cannula 14 so that it can close the tissue receiving opening 30, as shown in figure 1. When the inner cannula 16 slides over the tissue receiving opening 30, the annular cutting blade at the open distal end 32 thereof severs tissue prolapsing into the lumen of the outer cannula 14 through the tissue receiving opening 30. In certain embodiments, an introducer may be attached to the biopsy device. In those embodiments, the receiving opening 30 may include one or more beveled or radiused surfaces to prevent the introducer from catching on the sharp edges of the tissue receiving opening 30 and to facilitate smooth movement of the introducer as it is withdrawn over the tissue receiving opening 30. Various fluids (medication, anesthetic, saline, and/or air) are introduced into respective lumens of the outer and inner cannulas 14, 16 (e.g., via the manifold 18 and/or the outer cannula hub 20) to perform lavage of the biopsy site and/or facilitate movement of severed tissue samples through the inner cannula 16 and out the saline return fitting 26 via aspiration. Additional details regarding exemplary biopsy methods using a needle set 10 are described in U.S. patent number 10,022,110, which has been previously incorporated by reference herein.

The outer cannula hub 20 is coupled to a proximal end of the outer cannula 14. The inner cannula hub 22 is coupled to the inner cannula 16 between a midpoint and a proximal end thereof. The outer and inner cannula hubs 20, 22 are configured to be operatively coupled to corresponding components of a reusable portion (not shown), to thereby facilitate movement of the outer and inner cannulas 14, 16. The seal sleeve 24 disposed in the needle set 10 between the outer and inner cannula hubs 20, 22 to fix the minimum distance therebetween. The seal sleeve 24 is coupled to the outer cannula hub 20 and interferes with the inner cannula hub 22 (by way of a component on the reusable portion of the biopsy device to which the disposable needle set 10 is attached) to establish a lower limit on the distance between the outer and inner cannula hubs 20, 22. As such, the seal sleeve 24 also fixes the minimum distance between the respective distal ends of the outer and inner cannulas 14, 16. The saline return fitting 26 is configured to house a proximal end of the inner cannula 16 and to couple to and external vacuum source (not shown) for aspiration of severed tissue from the lumen of the inner cannula 16.

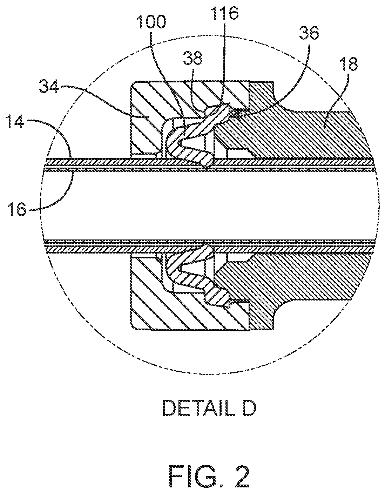

As described above, the inner cannula 16 is partially, slidably and coaxially disposed in the lumen of the outer cannula 14. The outer cannula 14 (and the inner cannula 16 disposed therein) are also partially and slidably disposed in the manifold 18 in the housing 12. When the outer cannula 14 slides longitudinally relative to the housing 12, it also slides longitudinally relative to the manifold 18, which is coupled to the housing 12. Because fluid passes through the manifold 18 to the respective lumens of the outer and inner cannulas 14, 16, the junction between the outer cannula 14 and the manifold 18 must be sealed/fluid-tight to prevent fluid leakage during operation of the needle set 10, which includes the outer cannula 14 sliding longitudinally relative to the manifold 18. One such fluidically active joint between the outer cannula 14 and the manifold 18 is labeled "D" in Figure 1 and shown in detail in Figure 2.

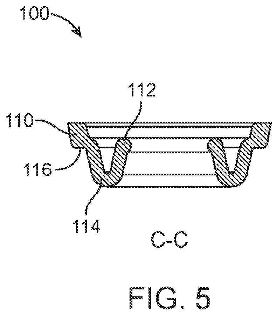

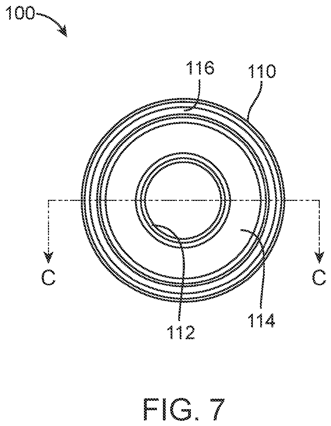

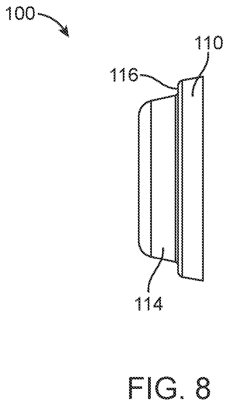

As shown in Figure 2, fluidically active joint D includes an outer cannula seal 100 configured to provide a fluid-tight seal between the outer cannula 14 and the distal end of the manifold 18 to prevent fluid leakage while allowing the outer cannula 14 to slide longitudinally relative to the manifold 18 during operation of the needle set 10. As shown in Figures 5-8, the outer cannula seal 100 includes an interference ring portion 110 on an outer diameter thereof. The interference ring portion 110 is configured to be captured by the distal end of the manifold 18 (as described below) to form an interference fit/seal therewith. The outer cannula seal 100 also includes a beaded ring portion 112 on an inner diameter thereof. The beaded ring portion 112 is configured to form a frictional fit/seal with an outer surface of the outer cannula 14 (as described below). The outer cannula seal 100 further includes a flexible portion/bellows feature 114 coupling the interference ring portion 110 to the beaded ring portion 112. The flexible portion 114 is configured to deform (e.g., bend, straighten and/or stretch) to thereby allow longitudinal movement of the outer cannula 14 relative to the manifold 18 while maintaining a fluid-tight seal between those two components. The outer cannula seal 100, including the interference ring portion 110, the beaded ring portion 112, and the flexible portion/bellows feature 114, are integrally formed as a single seal.

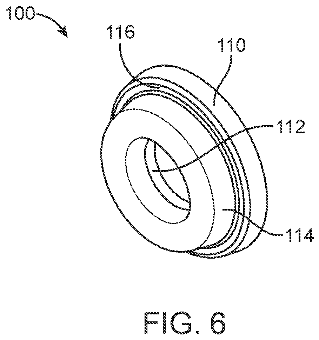

As shown in Figure 6, the outer cannula seal 100 has a partial conical shape. As shown in Figure 5, the outer cannula seal 100 (i.e., one wall thereof) has a V-shaped cross-section. The outer cannula seal 100 may be manufactured by mold forming a material such as ethylene propylene diene monomer ("EPDM") polymer. In one or more embodiments, the outer cannula seal 100 may be a high compliance seal (e.g., made from 70 Shore A EPDM).

The distal end of the manifold 18 includes a manifold cap 34 at a distal end thereof. During assembly, the distal end of the manifold 18 and the manifold cap 34 may be permanently coupled by laser and/or ultrasonic welding of the manifold cap 34 onto the distal end of the manifold 18 to permanently couple the outer cannula seal 100 to the distal end of the manifold 18. The distal end of the manifold 18 and the manifold cap 34 together define an annular space 36 adjacent the distal end of the manifold 18. As shown in Figure 2, the interference ring portion 110 (see Figure 5) is disposed in the annular space 36. Because of the relative sizes of the interference ring portion 110 and the annular space 36, the interference ring portion 110 is captured by an interference fit in the annular space 36 after the needle set 10 has been assembled. In particular, the manifold cap 34 defines a proximally facing annular lip 38, and the interference ring portion 110 defines a distally facing annular detent 36. The distally facing annular detent 36 is configured to interfere with a proximally facing annular lip 38 to prevent removal of the interference ring portion 110 from the annular space 36. As shown in Figure 5, the interference ring portion 110 has a cross-section that approximates a parallelogram. The sharp corners in the cross-section of the interference ring portion 110 increases interference with various surfaces of the annular space 36. The interference ring portion 110 is also compressed by the distal end of the manifold 18 and manifold cap 34 during assembly to strengthen the interference fit between the interference ring portion 110 and the distal end of the manifold 18, which generates a fluid-tight seal between these two components.

As shown in Figure 2, the beaded ring portion 112 (see Figure 5) is in contact with the outer surface of the outer cannula 14. As shown in Figure 5, the beaded ring portion 112 has a partially rounded cross-section, which increases friction between the beaded ring portion 112 and the outer surface of the outer cannula 14. The frictional fit between the beaded ring portion 112 and the outer cannula 14 generates a fluid-tight seal between these two components. The flexible portion 114 of the outer cannula seal 100 is biased to cause the beaded ring portion 112 to apply a force against the outer surface of the outer cannula 14 to create a fluid-tight seal between the beaded ring portion 112 and the outer surface of the outer cannula 14.

The flexible portion/bellows feature 116 is configured to deform (e.g., bend, straighten and/or stretch) to thereby allow longitudinal movement of the outer cannula 14 relative to the manifold 18 while maintaining a fluid-tight seal between those two components. The material from which the outer cannula seal 100 is formed (e.g. EPDM) facilitates the deformation of the flexible portion/bellows feature 116 with relative movement of the outer cannula 14 and the manifold 18. Accordingly, the outer cannula 14 can move longitudinally relative to the manifold 18 a predetermined distance without requiring movement between the beaded ring portion 112 and the outer cannula 14.

The interference fit between the interference ring portion 110 and the distal end of the manifold 18, the frictional fit between the beaded ring portion 112 and the outer cannula 14, and the deformation of the flexible portion/bellows feature 116 combined to form a fluid-tight seal between the outer cannula 14 and the distal end of the manifold 18. Accordingly, the outer cannula seal 100 allows longitudinal movement of the outer cannula relative to the manifold while maintaining a fluid-tight seal between the outer cannula and the distal end of the manifold.

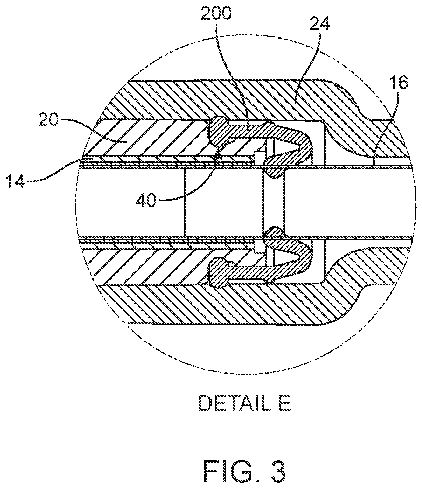

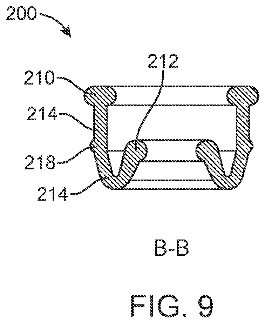

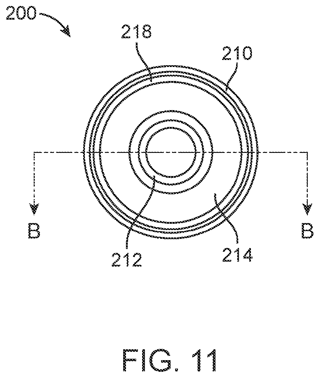

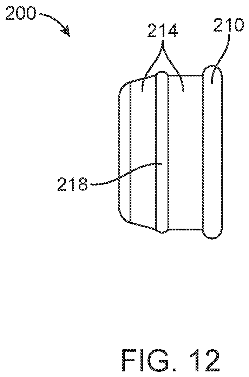

A fluidically active joint between the inner cannula 16 and the seal sleeve 24 is labeled "E" in Figure 1 and shown in detail in Figure 3. As shown in Figure 3, fluidically active joint E includes an inner cannula seal 200 configured to provide a fluid-tight seal between the inner cannula 16 and the seal sleeve 24 to prevent fluid leakage while allowing the inner cannula 16 to slide longitudinally relative to the seal sleeve 24 during operation of the needle set 10. As shown in Figures 9-12, the inner cannula seal 200 includes an interference ring portion 210 on an outer diameter thereof. The interference ring portion 210 is configured to be captured by the seal sleeve 24 (as described below) to form an interference fit/seal therewith. The inner cannula seal 200 also includes a beaded ring portion 212 on an inner diameter thereof. The beaded ring portion 212 is configured to form a frictional fit/seal with an outer surface of the inner cannula 16 (as described below). The inner cannula seal 200 further includes a flexible portion/bellows feature 214 coupling the interference ring portion 210 to the beaded ring portion 212. The flexible portion 214 is configured to deform (e.g., bend, straighten and/or stretch) to thereby allow longitudinal movement of the inner cannula 16 relative to the seal sleeve 24 while maintaining a fluid-tight seal between those two components. The flexible portion 214 of the inner cannula seal 200 is biased to cause the beaded ring portion 212 to apply a force against the outer surface of the inner cannula 16 to create a fluid-tight seal between the beaded ring portion 212 and the outer surface of the inner cannula 16.

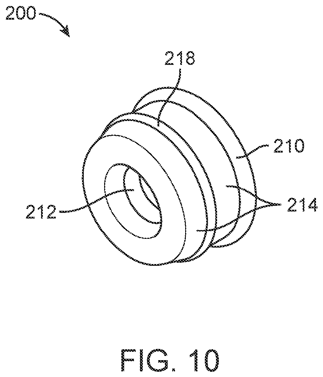

As shown in Figure 10, the inner cannula seal 200 has a partial conical shape. As shown in Figure 9, the inner cannula seal 200 (i.e., one wall thereof) has a J-shaped cross-section. The inner cannula seal 200 also includes a middle-beaded ring portion 218, which prevents an outer wall of the inner cannula seal 200 from buckling under stress. The inner cannula seal 200 may be manufactured by mold forming a material such as EPDM polymer. In one or more embodiments, the inner cannula seal 200 may be a high compliance seal (e.g., made from 70 Shore A EPDM).

The proximal end of the outer cannula hub 20 is disposed in the open distal end of the seal sleeve 24. The proximal end of the outer cannula hub 20 defines an annular groove 40 adjacent the distal end of the seal sleeve 24. As shown in Figure 3, the interference ring portion 210 (see Figure 9) is disposed in the annular groove 40. Because of the relative sizes of the interference ring portion 210 and the annular groove 40, the interference ring portion 210 is captured by an interference fit in the annular groove 40 after the needle set 10 has been assembled. In one or more embodiments, the seal sleeve 24 and the outer cannula hub 20 may be permanently coupled by an adhesive (or alternative means, such as laser and/or ultrasonic welding) to permanently couple the inner cannula seal 200 to the seal sleeve 24. As shown in Figure 9, the interference ring portion 210 has a cross-section that approximates a "bullnose" shape. The bullnose shape of the interference ring portion 210 is configured to interfere with various surfaces of the annular groove 40. The interference ring portion 210 is also compressed by the seal sleeve 24 and outer cannula hub 20 during assembly to strengthen the interference fit between the interference ring portion 210 and the seal sleeve 24, which generates a fluid-tight seal between these two components.

As shown in Figure 3, the beaded ring portion 212 (see Figure 9) is in contact with the outer surface of the inner cannula 16. As shown in Figure 9, the beaded ring portion 212 has a partially rounded cross-section, which increases friction between the beaded ring portion 212 and the outer surface of the inner cannula 16. The frictional fit between the beaded ring portion 212 and the inner cannula 16 generates a fluid-tight seal between these two components. The flexible portion 214 of the inner cannula seal 200 is biased to cause the beaded ring portion 212 to apply a force against the outer surface of the inner cannula 16 to create a fluid-tight seal between the beaded ring portion 212 and the outer surface of the inner cannula 16.

The flexible portion/bellows feature 214 is configured to deform (e.g., bend, straighten and/or stretch) to thereby allow longitudinal movement of the inner cannula 16 relative to the seal sleeve 24 while maintaining a fluid-tight seal between those two components. The material from which the inner cannula seal 200 is formed (e.g. EPDM) facilitates the deformation of the flexible portion/bellows feature 214 with relative movement of the inner cannula 16 and the seal sleeve 24. Accordingly, the inner cannula 16 can move longitudinally relative to the seal sleeve 24 a predetermined distance without requiring movement between the beaded ring portion 212 and the inner cannula 16.

The interference fit between the interference ring portion 210 and the seal sleeve 24, the frictional fit between the beaded ring portion 212 and the inner cannula 16, and the deformation of the flexible portion/bellows feature 214 combined to form a fluid-tight seal between the inner cannula 16 and the seal sleeve 24. Accordingly, the inner cannula seal 200 allows longitudinal movement of the inner cannula 16 relative to the seal sleeve 24 while maintaining a fluid-tight seal between the inner cannula 16 and the seal sleeve 24.

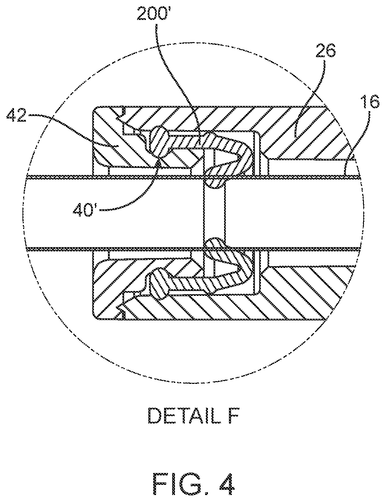

A fluidically active joint between the inner cannula 16 and the saline return fitting 26 is labeled "F" in Figure 1 and shown in detail in Figure 4. As shown in Figure 4, fluidically active joint F includes an inner cannula seal 200' configured to provide a fluid-tight seal between the inner cannula 16 and the saline return fitting 26 to prevent fluid leakage while allowing the inner cannula 16 to slide longitudinally relative to the saline return fitting 26 during operation of the needle set 10. The inner cannula seal 200' depicted in Figure 4 is identical to the inner cannula seal 200 depicted in Figure 3, which is described in detail above. The only difference between the inner cannula seals 200, 200'is their placement in the needle set 10, as shown in Figure 1.

The interference ring portion 210 on the inner cannula seal 200' is configured to be captured by the saline return fitting 26 (as described below) to form an interference fit/seal therewith. The saline return fitting 26 includes a saline return fitting cap 42 at a distal end thereof. The proximal end of the saline return fitting cap 42 defines an annular groove 40' adjacent the distal end of the saline return fitting 26. As shown in Figure 4, the interference ring portion 210 (see Figure 9) is disposed in the annular groove 40'. Because of the relative sizes of the interference ring portion 210 and the annular groove 40', the interference ring portion 210 is captured by an interference fit in the annular groove 40' after the needle set 10 has been assembled. In one or more embodiments, the saline return fitting 26 and the saline return fitting cap 42 may be permanently coupled by laser and/or ultrasonic welding to permanently couple the inner cannula seal 200 to the saline return fitting 26.

Similar to the inner cannula seal 200 depicted in Figure 3, the interference fit between the interference ring portion 210 and the saline return fitting 26, the frictional fit between the beaded ring portion 212 and the inner cannula 16, and the deformation of the flexible portion/bellows feature 214 combined to form a fluid-tight seal between the inner cannula 16 and the saline return fitting 26. Accordingly, the inner cannula seal 200' allows longitudinal movement of the inner cannula 16 relative to the saline return fitting 26 while maintaining a fluid-tight seal between the inner cannula 16 and the saline return fitting 26.

The fluidically active joints described above are configured to eliminate leakage by using high compliance seals and increased interference with the cannulas. Further, these seals reduce the frictional drag on the outer and/or inner cannulas which can improve firing speed and reduce cannula reciprocation forces. While fluidically active joints have been described between various components of the needle set 10, the needle set includes other fluidically active joints (e.g., between the outer cannula 14 and a proximal end of the manifold 18, as shown at "G" in Figure 1). Cannula seals similar to the outer and inner cannula seals 100, 200, 200' can be used in these fluidically active joints to generate fluid-tight seals while allowing relative movement of various components of the needle set 10. Also, various dimensions of the cannula seals may be modified to tailor the cannula seals for use in various locations in the needle set 10.

Although particular embodiments of the disclosed inventions have been shown and described, it is to be understood that the above description is provided for purposes of explanation and illustration only. Thus, various changes and modifications may be made without departing from the scope of the disclosed inventions. For example, not all of the components depicted and described in the disclosed embodiments are necessary, and various additional embodiments of the disclosed inventions may include any suitable combinations of the described components, and the general shapes and relative sizes of the components may be modified. While the systems and methods have been described with reference to needle sets for biopsy devices, embodiments can also be configured and utilized with any types of devices with fluidically active joints between components. Further, as will be appreciated by those with skill in the art that each of the individual variations described and illustrated herein has discrete components and features which may be readily separated from or combined with the features of any of the other several embodiments without departing from the scope or spirit of the present inventions. Accordingly, embodiments are intended to exemplify alternatives, modifications, and equivalents that may fall within the scope of the claims.

The following is a non-exhaustive list of embodiments of the invention that may be claimed in this application or in subsequently filed divisional applications:
Embodiment 1. A biopsy device, comprising:
   an elongated housing having a manifold;
   an outer cannula partially and slidably disposed in the manifold;
   an inner cannula partially and slidably disposed in a lumen of the outer cannula; and
   an outer cannula seal disposed between the manifold and the outer cannula, the outer cannula seal comprising
      an interference ring portion disposed adjacent an inner surface of the manifold,
      a beaded ring portion that contacts an outer surface of the outer cannula, and a flexible portion extending between the interference ring portion and the beaded ring portion.
Embodiment 2. The biopsy device of embodiment 1, wherein the outer cannula seal is configured to allow longitudinal movement of the outer cannula relative to the manifold while maintaining a fluid-tight seal therebetween.
Embodiment 3. The biopsy device of embodiment 1, wherein the outer cannula seal has a partial conical shape.
Embodiment 4. The biopsy device of embodiment 1, wherein the outer cannula seal has a V-shaped cross-section.
Embodiment 5. The biopsy device of embodiment 1, further comprising a manifold cap coupled to a distal end of the manifold, wherein the manifold cap and the manifold together define an annular space adjacent to the distal end of the manifold, and wherein the interference ring portion of the outer cannula seal is at least partially disposed in the annular space.
Embodiment 6. The biopsy device of embodiment 5, wherein the interference ring portion of the outer cannula seal forms an interference fit between the inner surface of the manifold and an inner surface of the manifold cap.
Embodiment 7. The biopsy device of embodiment 5, wherein the interference ring portion of the outer cannula seal defines a distally facing annular detent configured to engage a proximally facing annular lip of the manifold cap.
Embodiment 8. The biopsy device of embodiment 1, wherein the flexible portion of the outer cannula seal is biased to cause the beaded ring portion to apply a force against the outer surface of the outer cannula to create a fluid-tight seal between the beaded ring portion and the outer surface of the outer cannula.
Embodiment 9. The biopsy device of embodiment 1, wherein the flexible portion of the outer cannula seal is configured to allow the interference ring portion and beaded ring portion to move longitudinally relative to each other while maintaining a fluid-tight seal between the outer cannula and the manifold.
Embodiment 10. The biopsy device of embodiment 1, wherein the outer cannula seal comprises an ethylene propylene diene monomer ("EPDM") polymer.
Embodiment 11. The biopsy device of embodiment 1, wherein the outer cannula seal is manufactured using a mold-forming process.
Embodiment 12. A biopsy device, comprising:
   an elongated housing having a seal sleeve;
   an outer cannula;
   an outer cannula hub coupled to a proximal end of an outer cannula, the outer cannula hub being partially and slidably disposed in the seal sleeve;
   an inner cannula partially and slidably disposed in a lumen of the outer cannula; and
   an inner cannula seal disposed between the seal sleeve and the inner cannula, the inner cannula seal comprising
      an interference ring portion disposed adjacent an inner surface of the seal sleeve,
      a beaded ring portion that contacts an outer surface of the inner cannula, and
      a flexible portion extending between the interference ring portion and the beaded ring portion.
Embodiment 13. The biopsy device of embodiment 12, wherein the inner cannula seal is configured to allow longitudinal movement of the inner cannula relative to the seal sleeve while maintaining a fluidtight seal therebetween.
Embodiment 14. The biopsy device of embodiment 12, wherein the inner cannula seal has a partial conical shape.
Embodiment 15. The biopsy device of embodiment 12, wherein the inner cannula seal has a J-shaped cross-section.
Embodiment 16. The biopsy device of embodiment 12, wherein a proximal end of the outer cannula hub defines an annular groove adjacent a distal end of the seal sleeve, and wherein the interference ring portion of the inner cannula seal is at least partially disposed in the annular groove.
Embodiment 17. The biopsy device of embodiment 16, wherein the interference ring portion of the inner cannula seal forms an interfere fit within the annular groove.
Embodiment 18. The biopsy device of embodiment 12, wherein the inner cannula seal further comprises a middle beaded ring portion extending from on an outer surface of the flexible portion.
Embodiment 19. The biopsy device of embodiment 12, wherein the flexible portion of the inner cannula seal is biased to cause the beaded ring portion to apply a force against the outer surface of the inner cannula to create a fluid-tight seal between the beaded ring portion and the outer surface of the inner cannula.
Embodiment 20. The biopsy device of embodiment 12, wherein the flexible portion of the inner cannula seal is configured to allow the interference ring portion and the beaded ring portion to move longitudinally relative to each other while maintaining a fluid-tight seal between the inner cannula and the seal sleeve.
Embodiment 21. The biopsy device of embodiment 12, wherein the inner cannula seal comprises an ethylene propylene diene monomer ("EPDM") polymer.
Embodiment 22. The biopsy device of embodiment 12, wherein the inner cannula seal is manufactured using a mold-forming process.
Embodiment 23. A biopsy device, comprising:
   an elongated housing comprising a saline return fitting;
   an outer cannula partially and slidably disposed in the elongated housing;
   an inner cannula, wherein the inner cannula is partially and slidably disposed in each of a lumen of the outer cannula and a lumen of the saline return fitting; and
   an inner cannula seal disposed between an inner wall of the saline return fitting and the inner cannula, the inner cannula seal comprising
      an interference ring portion disposed adjacent an inner surface of the saline return fitting,
      a beaded ring portion that contacts an outer surface of the inner cannula, and
      a flexible portion that extends between the interference ring portion and the beaded ring portion.
Embodiment 24. The biopsy device of embodiment 23, wherein the inner cannula seal is configured to allow longitudinal movement of the inner cannula relative to the saline return fitting while maintaining a fluid-tight seal therebetween.
Embodiment 25. The biopsy device of embodiment 23, wherein the inner cannula seal has a partial conical shape.
Embodiment 26. The biopsy device of embodiment 23, wherein the inner cannula seal has a J-shaped cross-section.
Embodiment 27. The biopsy device of embodiment 23, further comprising a saline return fitting cap coupled to a distal end of the saline return fitting, wherein a proximal end of the saline return fitting cap defines an annular groove adjacent the distal end of the saline return fitting, and wherein the interference ring portion of the inner cannula seal is at least partially disposed in the annular groove.
Embodiment 28. The biopsy device of embodiment 27, wherein the interference ring portion of the inner cannula seal is at least partially disposed in the annular groove.
Embodiment 29. The biopsy device of embodiment 23, wherein the inner cannula seal further comprises a middle beaded ring portion extending from an outer surface of the flexible portion.
Embodiment 30. The biopsy device of embodiment 23, wherein the flexible portion of the inner cannula seal is biased to cause the beaded ring portion to apply a force against the outer surface of the inner cannula to create a fluid-tight seal between the beaded ring portion and the outer surface of the inner cannula.
Embodiment 31. The biopsy device of embodiment 23, wherein the flexible portion of the inner cannula seal is configured to allow the interference ring portion and the beaded ring portion to move longitudinally relative to each other while maintaining a fluid-tight seal between the inner cannula and the saline return fitting.
Embodiment 32. The biopsy device of embodiment 23, wherein the inner cannula seal comprises an ethylene propylene diene monomer ("EPDM") polymer.
Embodiment 33. The biopsy device of embodiment 23, wherein the inner cannula seal is manufactured using a mold forming process.

## Claims

1. A biopsy device, comprising:
an elongated housing having a seal sleeve;
an outer cannula;
an outer cannula hub coupled to a proximal end of an outer cannula, the outer cannula hub being partially and slidably disposed in the seal sleeve;
an inner cannula partially and slidably disposed in a lumen of the outer cannula; and
an inner cannula seal disposed between the seal sleeve and the inner cannula, the inner cannula seal comprising
an interference ring portion disposed adjacent an inner surface of the seal sleeve,
a first beaded ring portion that contacts an outer surface of the inner cannula, and
a flexible portion extending between the interference ring portion and the first beaded ring portion.

2. The biopsy device of claim 1, wherein the inner cannula seal is configured to allow longitudinal movement of the inner cannula relative to the seal sleeve while maintaining a fluid-tight seal therebetween.

3. The biopsy device of claim 1 or claim 2, wherein the inner cannula seal has a partial conical shape.

4. The biopsy device of any preceding claim, wherein the inner cannula seal has a J-shaped cross-section.

5. The biopsy device of any preceding claim, wherein a proximal end of the outer cannula hub defines an annular groove adjacent a distal end of the seal sleeve, and wherein the interference ring portion of the inner cannula seal is at least partially disposed in the annular groove.

6. The biopsy device of claim 5, wherein the interference ring portion of the inner cannula seal forms an interference fit within the annular groove.

7. The biopsy device of claim 5 or 6, wherein the interference ring portion comprises a rounded or bullnose cross-sectional profile configured to enhance interference with the annular groove.

8. The biopsy device of any of preceding claims 5 to 7, wherein the annular groove is configured to axially retain the interference ring portion against proximal and distal displacement.

9. The biopsy device of any preceding claim, wherein the inner cannula seal further comprises a middle beaded ring portion extending from an outer surface of the flexible portion.

10. The biopsy device of claim 9, wherein the middle beaded ring portion is configured to stabilize the flexible portion against radial buckling during axial compression.

11. The biopsy device of any preceding claim, wherein the flexible portion of the inner cannula seal is biased to cause the first beaded ring portion to apply a force against the outer surface of the inner cannula to create a fluid-tight seal between the first beaded ring portion and the outer surface of the inner cannula.

12. The biopsy device of any preceding claim, wherein the flexible portion of the inner cannula seal is configured to allow the interference ring portion and the first beaded ring portion to move longitudinally relative to each other while maintaining a fluid-tight seal between the inner cannula and the seal sleeve.

13. The biopsy device of any preceding claim, wherein the flexible portion comprises a bellows structure.

14. The biopsy device of any preceding claim, wherein the first beaded ring portion has a partially rounded cross-sectional profile to increase localized sealing pressure against the outer surface of the inner cannula.

15. The biopsy device of any preceding claim, wherein the interference ring portion is compressed between the seal sleeve and the outer cannula hub to increase sealing integrity.
